## Europäisches Patentamt
## European Patent Office
## Office européen des brevets

(19)

(11) Veröffentlichungsnummer: **0 054 785**
**B1**

(12) **EUROPÄISCHE PATENTSCHRIFT**

(45) Veröffentlichungstag der Patentschrift:
03.07.85

(21) Anmeldenummer: 81110135.1

(22) Anmeldetag: 04.12.81

(51) Int. Cl.⁴: **A 61 C 5/10**, A 61 C 9/00,
A 61 C 19/04, A 61 F 2/00

(54) Verfahren zur Herstellung medizinischer und zahntechnischer alloplastischer, endo- und exoprothetischer Passkörper.

(30) Priorität: 24.12.80 CH 9561/80

(43) Veröffentlichungstag der Anmeldung:
30.06.82 Patentblatt 82/26

(45) Bekanntmachung des Hinweises auf die Patenterteilung:
03.07.85 Patentblatt 85/27

(84) Benannte Vertragsstaaten:
AT BE CH DE FR GB IT LI NL SE

(56) Entgegenhaltungen:
EP - A - 0 025 911
EP - A - 0 033 492
EP - A - 0 040 165
FR - A - 1 457 269
US - A - 3 777 740
US - A - 3 861 044
US - A - 4 182 312

(73) Patentinhaber: Mörmann, Werner H., Dr.med.dent.,
Hofstrasse 104, CH-8044 Zürich (CH)
Patentinhaber: Brandestini, Marco, Dr.sc.techn.,
Gartenstrasse 10, CH-8702 Zollikon (CH)

(72) Erfinder: Mörmann, Werner H., Dr.med.dent.,
Hofstrasse 104, CH-8044 Zürich (CH)
Erfinder: Brandestini, Marco, Dr.sc.techn.,
Gartenstrasse 10, CH-8702 Zollikon (CH)

(74) Vertreter: Werner, Georges, E. Blum & Co,
Patentanwälte Vorderberg 11, CH-8044 Zürich (CH)

ACTORUM AG

**Beschreibung**

Die Erfindung betrifft ein Verfahren zur Herstellung medizinischer oder zahnmedizinischer alloplastischer Passkörper.

Bei der Inkorporation alloplastischer Implantate spielt die exakte Passung zur Herstellung einer guten Verankerung und dauerhaftem Sitz für die physiologische Belastung der aufnehmenden Gewebe aber auch für die ausgeglichene physikalische Belastung der Alloplastik und/oder ihre Funktion eine entscheidende Rolle. Dies gilt beispielsweise für enossale und alveoläre Implantate im Kieferbereich, für exoprothetische, optische Augenhaftschalen, für Hüftgelenksendoprothesen, aber auch für rein zahnärztliche alloplastische, endo- und exoprothetische Passkörper, wie Zahnfüllungen oder Kronen und Brükken. Bei Knochenimplantaten und den erwähnten Sehhilfen finden üblicherweise konfektionierte Passkörper Verwendung, die entweder nach individuellen Massen ausgewählt werden oder das zu versorgende Organ wird nach Konfektionsmassen gestaltet.

In der Zahnheilkunde dominiert die individuelle Gestaltung von Passkörpern, die nach überlieferten Prinzipien der Zahntechnik hergestellt werden. Die Anwendung des genannten Verfahrens im zahnärztlichen Bereich wird im folgenden beispielsweise aufgeführt:

Zahnärztliche Füllungen aller Art sind als alloplastische (= Fremdkörper) Implantate anzusehen, deren Zweck es ist, den Zahn als Organ, insbesondere die Zahnkrone in Form und Funktion dauerhaft wiederherzustellen. Bekannt sind Amalgam-, Metallguss (Gold)-, Keramik- und Kunststoffkompositfüllungen.

Mit Hilfe konventioneller Zahntechnik können Einlagefüllungen in Form von Inlays, Onlays und Overlays in bezug auf Materialbeständigkeit, physikalische Eigenschaften und Morphologie, befriedigend hergestellt werden; nachteilig sind die hohen Materialkosten z.B. Gold. Zudem erfordert die konventionelle zahntechnische Herstellung von Einlagefüllungen, sei es aus Metall oder bei Verwendung anderer Materialien, beispielsweise Komposit oder Keramik, eine Vielzahl technischer Abläufe, wie Abdrucknahme, Modellherstellung, Wachsmodellation, Einbetten des Wachsmodells, Metallguss bzw. Kunststoffspritzguss oder Stopfen, Pressen und Heiss-Polymerisation.

Im US-Patent 4 182 312 wurde vorgeschlagen, dreidimensionale Oberflächeninformationen von Zähnen und umliegendem Gewebe direkt am Patienten mechanisch abzutasten, um damit, wie bei einer Kopierfräsmaschine, Bearbeitungsmaschinen zur Herstellung von Zahnprothesen zu steuern. Hierzu muss eine Sonde mit einem daran fest fixierten Übertragungsgestänge, in Kontakt mit den interessierenden Oberflächen, vom Zahnarzt im Munde des Patienten geführt werden. Nachteilig ist dabei, dass zur vollständigen Registrierung einer Fläche sehr viele Abtastbewegungen durchzuführen sind, was zeitraubend und für die Patienten belastend ist. Umständlich ist auch, dass die Sondenspitzen, je nach der Form des erforderlichen Bearbeitungswerkzeuges, gewechselt werden müssen. Zudem wird eine fixe Bezugsebene zwischen Bearbeitungswerkzeug und Abtastorgan benötigt. Nachteilig und für den Patienten unangenehm ist, dass bei dieser Art der Abtastung die Registriereinheit mit Hilfe eines Trays am Kiefer des Patienten mit Gips befestigt werden muss. Durch die Befestigung dieser umfangreichen Hilfseinrichtungen am Patienten wird der Zugang für ungestörte Abtastungen mit der Sonde behindert. Diese umfangreichen Hilfsmassnahmen direkt am und im Munde des Patienten sind vom zahnärztlichen Standpunkt aus als wesentlich stärkere Belastung für den Patienten anzusehen als die herkömmlichen Abformungs- und Herstellungsmethoden von Zahnprothesen.

Neben der im obigen US-Patent 4 182 312 beschriebenen mechanischen Abtastmethode sind auch optische 3-dimensionale Messvorrichtungen bekannt (Butcher, G.W. et al., Brit. dent. J., 1981, 151, p. 304; Mikhail, E.M. chap. 17–19 of «Photogrammetry»; F.H. Moffit ed. pp. 579–582, Harper & Row, New York 1980).

Diesen ist gemeinsam, dass sie ausschliesslich diagnostischen Zwecken dienen. Die Vorrichtungen von Butcher und Mikhail sind nichtautomatische Instrumente, die eine zeitaufwendige, manuelle Koordinatenbestimmung erfordern.

Die Moiré-Topographie liefert eine Technik zur automatischen Profilaufnahme, beschränkt sich jedoch im Artikel von K. Takasaki («Moiré Topography, Systems and Applications» chap. 8 in Handbook of Non-topographic Photogrammetry, H.M. Karara, ed., Am. soc. of Photogrammetry everybody press 1979) auf eine grob abgestufte, nicht absolute Bildtiefenbestimmung.

Im US-Patent 3 861 044 ist in allgemeiner Form ein Verfahren beschrieben, bei welchem ein Passkörper zum Einsetzen in eine Ausnehmung eines Zahnes herzustellen ist. Dabei wird die Form der Ausnehmung photographisch erfasst und Daten der Aufnahme werden gespeichert. Anschliessend wird die Ausnehmung des Zahnes mit Wachs gefüllt, der Wachsfüllkörper vom Zahnarzt in die gewünschte Endform gebracht und dann eine neue Aufnahme gemacht, um deren Daten ebenfalls zu speichern. Die Gesamtheit der Daten wird dann zur Steuerung einer Werkzeugmaschine verwendet und der definitive Füllkörper hergestellt.

Beim bekannten Verfahren muss somit eine Art provisorischer Füllkörper in einem Zwischenschritt hergestellt werden. Demgegenüber erlaubt das erfindungsgemässe Verfahren die Herstellung eines endgültigen Füllkörpers mit einer einzigen Aufnahme, welche mittels vorprogrammierter Daten zu einem vollständigen Konstruktionsplan verarbeitet wird, ohne das ein provisorischer Füllkörper und eine zweite photographische Erfassung nötig wäre.

Die europäische Patentanmeldung EP-A-0040165 bezieht sich auf ein Verfahren, welches jenem der vorliegenden Erfindung in groben Zügen nahekommt, ohne jedoch das wichtigste Merkmal, nämlich die datenmässige Vervollständigung des Konstruktionsplanes in einer für den Fachmann verständlichen Weise aufzuzeigen. Es sei noch ausdrücklich darauf verwiesen, dass dieses Dokument für die Ansprüche 1,2 und 4–6 unter Art. 54 (3) EPUe fällt, jedoch für die Frage der erfinderischen Tätigkeit dieser Ansprüche nicht von Bedeutung ist.

In der nachstehend näher erläuterten, erfindungsgemässen Lösung werden die bekannten umständlichen Techniken umgangen.

Aufgabe der Erfindung ist, z.B. Füllungskörper im Sinne von Einlagefüllungen aus einem fabrikmässig vorbereiteten Füllungsmaterial als Passkörper aus Hartmaterial, wie Metall, Keramik, Kunststoff, Kompositkunststoff oder Kombinationen derselben, unter Umgehung konventioneller Zahntechnik, nach individuellen Kavitätenmassen auszuarbeiten.

Erfindungsgemäss wird die Aufgabe bei einem Verfahren zur Herstellung medizinischer oder zahnmedizinischer alloplastischer, endo- und exoprothetischer individueller Passkörper aus Hartmaterialien dadurch gelöst, dass Oberflächen von Körperorganen oder an Körperorganen geschaffene Oberflächen, körperhafte Formen und Räume in mindestens Teilen ihrer Wandungen und ihren Begrenzungen und die an die vorgenannten Gestaltungen angrenzenden, nicht operativ bearbeiteten Oberflächen in ihrer räumlichen und topographischen Gestalt auf optischem Weg berührungsfrei räumlich erfasst und mittels 3-dimensionaler Bildauswertung registriert werden und anhand des Registrates ein Konstruktionsplan des zur Organergänzung erforderlichen Passkörpers berechnet und zur Kontrolle bildlich dargestellt wird, wobei zur Erstellung dieses Konstruktionsplanes optisch nicht erfasste Flächen durch Anpassung vorprogrammierter Krümmungradien an die Grenzlinien der erfassten Flächen ergänzt werden, und dass schliesslich das bestimmungsgemässe Material durch den Konstruktionsplan und ein Bearbeitungsprogramm zu einem der Form des betreffenden Organs zumindest annähernd angepassten alloplastischen Körper gestaltet wird.

Bevorzugte Ausgestaltungen des erfindungsgemässen Verfahrens gehen aus den Ansprüchen 2 bis 6 hervor.

Unter Oberflächen, an die der Passkörper anpassbar ist, werden alle Gewebe-Organoberflächen des menschlichen Körpers verstanden, so u.a. auch Weich- oder Hartgewebe oder Körpersubstanzen, die kein Gewebe im eigentlichen Sinne sind, z.B. Zahnschmelz.

Nachstehend werden Ausführungsbeispiele näher erläutert.

Es zeigen

Fig. 1 eine schematische Darstellung des Ablaufes des erfindungsgemässen Verfahrens,

Fig. 2 eine schaubildliche und schematische Ansicht einer natürlichen Zahnkrone mit einer Inlaykavitäten-Präparation,

Fig. 3 eine Draufsicht auf eine Teil-Zahnreihe und

Fig. 4 eine Teilschnittansicht durch eine Zahnreihe mit Andeutung der Lage des Bildaufnahmekopfes.

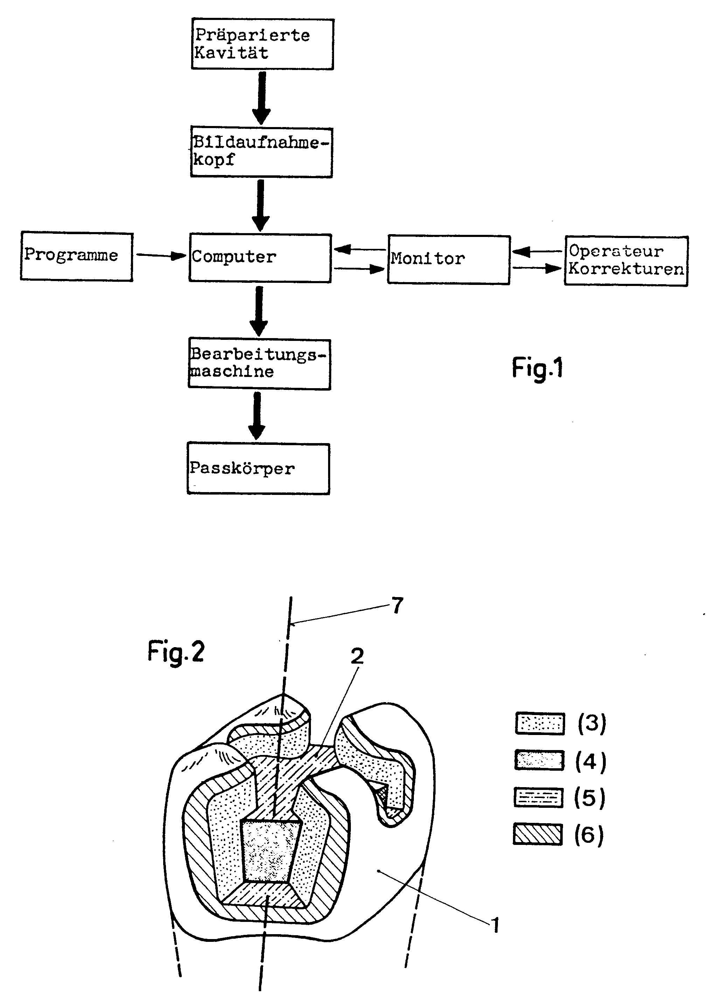

Nach der schematischen Darstellung in Fig. 1 wird eine bildliche Erfassungsmethode, sei es optisch oder elektronenoptisch, dazu benutzt, beispielsweise eine Zahnkavität direkt in der Mundhöhle des Patienten oder ausserhalb der Mundhöhle, an einem Abdruck der Kavität, durch Positionieren eines Bildaufnahmekopfes in ihrer Geometrie, ihren Grenzlinien und Nachbarbeziehungen zu erfassen und diese Information an einen programmgesteuerten Computer zur Berechnung einer 3-dimensionalen Konstruktionsdarstellung zu übermitteln. Die Computerdarstellung der Zahnkavität wird anhand eines Bildschirmes vom Zahnarzt auf Vollständigkeit in bezug auf die operationellen Anforderungen überprüft und gegebenenfalls eine günstigere Positionierung des Bildaufnahmekopfes oder operative Änderungen bzw. Ergänzungen der Präparation am Zahn vorgenommen. Die für richtig befundene Computerdarstellung der Zahnkavität wird anhand der Nachbar- und Grenzstrukturen durch Extrapolationen des Computers und nötigenfalls durch direkte Konstruktionsangaben des Zahnarztes, beispielsweise Tastatur, Lichtgriffel oder Digitalisierer, in ihren fehlenden äusseren Zahnflächen so ergänzt, dass sie einen vollständigen, den Zahn in allen Teilen wiederherstellenden Passkörper ergibt. Nach Abschluss der Konstruktion steuert der Computer anhand der konstruktionsgegebenen Daten und mit einem Steuerpogramm die Bearbeitung zur Herstellung des Füllungspasskörpers aus einem vordimensionierten Füllungsrohmaterial. Dadurch kann, unter Umgehung von herkömmlichen, labortechnischen Arbeitsmethoden, ein individueller Passkörper rationell ausserhalb der Mundhöhle hergestellt werden, der dem Zahnarzt sofort zur Inkorporation zur Verfügung steht.

Anhand der Fig. 2 bis 4 wird die Situation bei der Anwendung des erfindungsgemässen Verfahrens im zahnärztlichen Bereich beispielsweise erläutert.

Fig. 2 zeigt die Ansicht einer natürlichen Zahnkrone 1 mit einer typischen, sich auf mehrere Zahnflächen erstreckenden Zahnkavität 2, deren axiale Wände 3, 4 in jedem Teil mit einem nach okklusal, d.h. zur Kaufläche offenen Winkel von mindestens 4°, zu einer gemeinsamen Einschubachse 7 geneigt sind. Den Kavitätenboden bilden horizontale Flächen 5 und der Kavitätenrand wird durch eine Schrägung 6 gebildet.

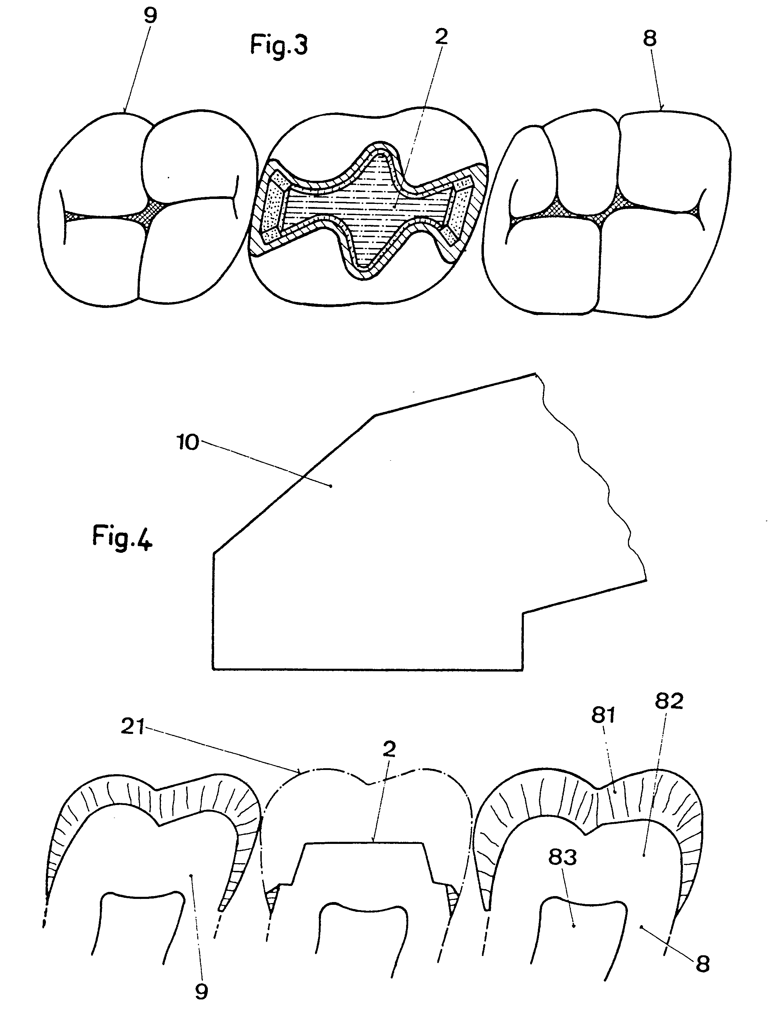

Fig. 3 zeigt die Okklusalansicht einer in allen Wandteilen nach okklusal offenen dreiflächigen, mesialokklusal-distalen Inlaykavität 2, mit mesialem 8 und distalem 9 Nachbarzahn.

Fig. 4 zeigt die Darstellung eines mesio-distalen, mittigen Schnittprofils der abzubildenden Zahnkavität 2, mit mesialem 8 und distalem 9 Nachbarzahn und Umriss des Bildaufnahmekopfes 10. An den Zähnen ist die äussere Schmelzschicht 81, das Dentin 82, das Pulpakavum 83, erkennbar. Die Linie 21 markiert den Umriss des herzustellenden Passkörpers.

Vorgängig der bildlichen Erfassung der Oberflächen der zu versorgenden Körperorgane oder der mit einer Abdruckmasse hergestellten Abformungen, können deren Flächen mindestens teilweise mit einer abbildungstechnischen Beschichtung versehen werden. Das zu erfassende Bild wird durch die Beschichtung verstärkt oder differenziert bzw. kontrastiert, und es können störende Strukturen abgedeckt werden, was die bildhafte Erfassung erleichtert.

Die Erfindung stellt beispielsweise dem Zahnarzt, unter Ersatz der klassischen Abformung und labortechnischen Füllungsherstellung, ein Verfahren zur Verfügung, welches die entsprechend einer Inlaypräparation ausgearbeitete Kavität räumlich dimensionsgetreu erfasst, speichert und zur Kontrolle bildlich darstellt. Die in der zahnärztlichen Praxis oder in einem Labor vorhandene Bearbeitungsmaschine fertigt den Passkörper beispielsweise spanabhebend, schleifend oder erodierend gemäss den gespeicherten Angaben direkt aus einem Rohling. Der Passkörper steht dann dem Zahnarzt für die Zahnbehandlung unmittelbar zur Verfügung.

Das beschriebene Verfahren ermöglicht es, durch kombinierte Anwendung von in der Technik bereits angewandten Vermessungs-, Aufzeichnungs-, Darstellungs- und Berechnungsmethoden im medizinisch-zahntechnischen Bereich, Alloplastiken, unter optimierten, material- und arbeitstechnischen Bedinungen herzustellen. Dadurch wird eine Qualitätssteigerung und Kostensenkung erzielt.

**Patentansprüche**

1. Verfahren zur Herstellung medizinischer oder zahnmedizinischer alloplastischer, endo- und exoprothetischer individueller Passkörper aus Hartmaterialien, bei welchem Oberflächen von Körperorganen oder an Körperorganen geschaffene Oberflächen, körperhafte Formen und Räume in wenigstens Teilen ihrer Wandungen und ihren Begrenzungen und die an die vorgenannten Gestaltungen angrenzenden, nicht operativ bearbeiteten Oberflächen in ihrer räumlichen und topographischen Gestalt auf optischem Weg berührungsfrei räumlich erfasst und mittels 3-dimensionaler Bildauswertung registriert werden und anhand des Registrates ein Konstruktionsplan des zur Organergänzung erforderlichen Passkörpers berechnet und zur Kontrolle bildlich dargestellt wird, wobei zur Erstellung dieses Konstruktionsplanes optisch nicht erfasste Flächen durch Anpassung vorprogrammierter Krümmungsradien an die Grenzlinien der erfassten Flächen ergänzt werden, und bei welchem schliesslich das bestimmungsgemässe Material durch den Konstruktionsplan und ein Bearbeitungsprogramm zu einem der Form des betreffenden Organs zumindest annähernd angepassten alloplastischen Körper gestaltet wird.

2. Verfahren nach Anspruch 1, bei dem die aufgenommenen Oberflächeninformationen bzw. Konstruktionsdaten auf einem Monitor bildlich dargestellt werden, wonach ein Rechner die in oder auf die erfasste Form passende Alloplastik berechnet und ebenfalls bildlich darstellt, wobei vom Operateur direkt konstruktive Korrektur- bzw. Ergänzungsanweisungen eingegeben werden können, bis die Konstruktion den endgültigen Erfordernissen entspricht, und die berechneten Konstruktionsdaten dann an die Steuerungseinheit einer Bearbeitungsmaschine weitergegeben werden.

3. Verfahren nach einem der Ansprüche 1 bis 2, bei dem vorgängig der bildlichen Erfassung der Oberflächen der Körperorgane oder der davon mit Abdruckmassen hergestellten Abformungen diese Flächen mindestens teilweise mit einer abbildungstechnischen Beschichtung versehen werden.

4. Verfahren nach einem der Ansprüche 1 bis 3, bei dem die dem alloplastisch zu versorgenden Organ benachbarten und funktionell antagonistischen Organe in ihrer Oberflächengestalt, räumlichen Orientierung und ihrem Abstand erfasst werden, und die zu fertigenden alloplastischen Passstücke, zumindest bis auf eine eventuelle intraoperativ manuell vorzunehmende Anpassung, entsprechend den Nachbar- und Antagonistenbeziehungen geformt werden.

5. Verfahren nach einem der Ansprüche 1 bis 4, bei dem eine Steuerungsvorrichtung für Bearbeitungsmaschinen verwendet und zusätzlich so programmiert wird, dass auf wenigstens Teilen der Passfläche eine Unterdimensionierung hergestellt wird, wodurch zwischen den Alloplastikstücken und der erfassten Innen- oder Aussenform des zu ergänzenden Organs, Raum zur Verfügung gestellt wird, in welchem ein zur Anpassung oder Haftungsvermittlung der Alloplastik mit dem Gewebe erforderliches Material oder eine aus physiologischen Gründen erforderliche Zwischensubstanz ihren Platz findet, oder dass, je nach Anwendung, aus technischen oder funktionellen Erfordernissen das Steuerungsprogramm wenigstens eine teilweise Überdimensionierung der Alloplastik vornimmt.

6. Verfahren nach Anspruch 5, bei dem die Steuerungsvorrichtung zur Gestaltung der äusseren Alloplastikoberfläche zusätzlich so programmiert wird, dass diese im gesamten Grenzbereich zumindest annähernd als Fortsetzung der Organaussenflächen gestaltet wird, wobei je nach Erfordernis form- und/oder funktionsbedingte, vorgegebene Masse berücksichtigt werden.

**Claims**

1. Process for the manufacture of individually shaped medical and dental, alloplastic, endoprosthetic and exoprosthetic fittings made of hard materials, wherein the surfaces of organs of the body or surfaces created at organs of the body, three dimensional forms and spaces along at least part of their walls and boundaries and operatively non treated surfaces adjacent to those configurations are optically detected in a contact-free manner with respect to their topographical and spatial characteristic and then are recorded by means of 3-dimensional image analysis, wherein by means of this record a design plan for a fitting suited for restoring said organ is calculated and graphically displayed for a visial check, wherein when making this design plan those surfaces which were not optically detected are completed by adapting pre-programmed curvatures to the boundaries of detected surfaces and wherein finally the respective material is shaped on the basis of said design plan and of a machining programme to an alloplastic fitting, the shape of which is adapted at least approximately to the shape of said organ.

2. Process as defined in claim 1, wherein the recorded surface information and design data, respectively, are displayed on a monitor, according to which data a calculator calculates and displays the alloplastic fitting matching into or onto the recorded shape and wherein instructions for constructive corrections or completions directly can be introduced by the operator until the design corresponds to the final requirements, whereafter the calculated design data are transmitted to the control unit of a processing machine.

3. Process as defined in one of the claims 1 to 2, wherein before performing the image detection of the surfaces of the organs of the body or of the molds made thereof by an impression material said surfaces are at least partly provided with an image coating.

4. Process as defined in one of the claims 1 to 3, wherein the organs neighboring and functionally antagonistic to the organ to be alloplastically restored are detected with respect to their orientation in space and to their distance and wherein the alloplastic fittings are shaped according to the respective neighboring and antagonistic relation with the possible exception of intraoperative manually performed adaptions.

5. Process as defined in one of the claims 1 to 4, wherein a control device for a processing machine is used and is further programmed in such a way that at least on portions of the fitting surface reduced dimensions are produced, whereby between the alloplastic fittings and the detected inner or outer form of the organ to be restored a clearance is provided for a material necessary for the adaption or adhesion of the alloplastic fitting to the tissue or for an intermediate substance necessary for physiological reasons or in such a way that depending on the application the control programme at least partly produces larger dimensions for technical or functional reasons.

6. Process as defined in claim 5, wherein the control device for shaping the outer surface of the alloplastic fitting is further programmed in such a way that this surface in its whole boundary region is shaped at least approximately as a continuation of the outer surface of the organ, wherein according to individual requirements given dimensions are used depending on aspects of its form or its function.

**Revendications**

1. Procédé de réalisation d'éléments rapportés individuels médicaux et dentaires, alloplastique, endoprothétiques et exoprothétiques en des matériaux durs, dans lequel des surfaces d'organes du corps ou des surfaces formées sur les organes du corps, des formes corporelles et des espaces, sont explorés dans l'espace et sans contact par voie photographique dans au moins des parties de leurs parois et de leurs limites ainsi que les surfaces voisines des formes qui viennent d'être indiquées et qui ne sont pas façonnées opérationnellement dans leur forme dans l'espace et dans leur forme topographique, et sont enregistrées au moyen d'une évaluation tridimensionnelle de l'image, un plan d'exécution de l'élément rapporté nécessaire pour compléter l'organe étant calculé à partir des données enregistrées et présenté sous forme d'une image en vue d'un contrôle, les surfaces qui ne sont pas détectées optiquement étant complétées par raccordement de surfaces à degrés de courbure préprogrammés aux lignes limites des surfaces détectées en vue de la réalisation de ce plan d'exécution, et le matériau approprié est finalement conformé au moyen du plan d'exécution et d'un programme de façonnage en un corps alloplastique adapté au moins approximativement à la forme de l'organe concerné.

2. Procédé selon la revendication 1, dans lequel les informations de surface ou les données d'exécution saisies sont représentées sous forme d'images sur un moniteur, après quoi une calculatrice calcule l'élément alloplastique à disposer dans ou sur la forme détectée lequel est également représenté sous forme d'image, des indications de correction ou de complément d'exécution pouvant être fournies directement par l'opérateur jusqu'à ce que l'exécution corresponde finalement aux besoins, et les données calculées de la forme d'exécution étant alors transmises à l'unité de commande d'une machine de façonnage.

3. Procédé selon la revendication 1 ou 2, dans lequel, avant la saisie figurative des surfaces des organes du corps ou de déformations de surfaces provoquées dans les masses pour empreintes, ces surfaces sont munies au moins partiellement d'une couche favorisant la prise de vue.

4. Procédé selon l'une des revendications 1 à 3, dans lequel les organes voisins et fonctionnellement antagonistes de l'organe à traiter alloplastiquement sont détectés sur le plan de la forme de leur surface, de leur orientation dans l'espace et de leurs dimensions, et les pièces rapportées alloplastiques à réaliser sont conformées, du moins jusqu'à une adaptation intermédiaire éventuelle à effectuer manuellement, conformément aux relations de voisinage et antagonistes.

5. Procédé selon l'une des revendications 1 à 4, dans lequel un dispositif de commande est utilisé pour les machines de façonnage et est de plus programmé de manière que soit réalisé un sous-dimensionnement sur au moins des parties des surfaces d'adaptation, l'espace existant entre l'élément alloplastique et la forme interne ou externe de l'organe à compléter qui a été saisie étant destiné à recevoir le matériau nécessaire à l'adaptation ou à l'adhérence de l'élément alloplastique sur le tissu ou une substance intermédiaire nécessaire pour des raisons physiologiques, ou bien de manière qu'en fonction de l'utilisation, soit assuré un sur-dimensionnement au moins partiel de l'élément alloplastique pour des raisons techniques ou fonctionnelles du programme de commande.

6. Procédé selon la revendication 5, dans lequel le dispositif de commande destiné à la conformation de la surface extérieure de l'élément alloplastique est en outre programmé de manière que cette surface soit conformée dans la totalité de sa zone limite pour constituer au moins approximativement un prolongement de la surface externe de l'organe, en tenant compte, selon les besoins, d'une dimension préalable déterminée par la forme et/ou la fonction.

Fig.1

Fig.2

(3)
(4)
(5)
(6)

Fig.3

Fig.4